# EUROPEAN PATENT SPECIFICATION

(11) **EP 2 048 852 B1**
(45) Date of publication and mention of the grant of the patent: **27.01.2016**
(21) Application number: 07118058.2
(22) Date of filing: 08.10.2007
(51) Int. Cl.: H04L 29/08, G06F 17/30, A61B 5/00

(54) **Service aggregation system and method**
Dienstaggregationssystem und Verfahren
Système et procédé d'agrégation de services

(43) Date of publication of application: 15.04.2009
(73) Proprietor: Fifthplay NV, 9100 Sint-Niklaas (BE)
(72) Inventor: Baekelmans, John, 2550, Kontich (BE); Vos, Thibaut, 2650, Edegem (BE)
(74) Representative: D'Halleweyn, Nele Veerle Trees Gertrudis

(56) References cited:
- US-A1- 2004 102 683
- US-A1- 2004 267 927
- US-A1- 2006 089 990
- US-A1- 2007 067 265
- US-B1- 6 401 085
- SAHA A: "Application Framework for e-business: Portals" INTERNET CITATION, [Online] XP002276158 Retrieved from the Internet: URL:ftp://www6.software.ibm.com/software/d eveloper/library/portals.pdf> [retrieved on 2004-04-05]

## Description

### Field of the Invention

The present invention generally relates to service delivery, i.e. technology enabling end-customers to consume at home services from so called service providers or service partners. More particularly, the invention relates to aggregating services from multiple service providers enabling delivery of services to a large amount of end-customers, in a many-to-many relationship and in a consistent, uniform fashion.

### Background of the Invention

Nowadays, service delivery typically relies on client-server architectures and dedicated, service-specific terminals at the customer end.

In the field of telemedicine for instance, International Patent Application WO 03/050642, entitled "A Visual Medical Monitoring System for a Remote Subject", describes a system for a remote medical support service consisting of a dedicated end-customer terminal with videoconference functionality, and a medical service centre. The remote end-customer terminal and the medical service centre support bidirectional client-server type of communication over telephone wiring, enabling supervision of a patient at home, measuring and transferring medical data such as heart rate, weight gain, blood pressure, etc., and audio/video communication with medical personnel. The remote client features monitoring equipment, for instance integrated in a wristband, and a remote gateway that automatically initiates a dial-up or TCP/IP connection towards the medical service centre. At the medical service centre, a central gateway may be foreseen for relaying/switching the communication.

The system described in WO 03/050642 supports a single service and relies on dedicated, service-specific end-customer equipment that communicates with the service partner's infrastructure in a client-server mode.

A similar service is described in European Patent Application EP 1 726 257 entitled "In-Home Patient Monitoring System". In this system, remote monitoring of plural patients is made possible from a centralized location through remote monitoring units with dual communication mode (wired/wireless) and voice processing function. The centralized location is hosting the server that collects the vital sign data from the remote monitoring units through client-server communication. The central server is made accessible by workstations through web-based communication.

The system described in EP 1 726 257 also requires a dedicated, service-specific remote unit to be used by every patient. Further, EP 1 726 257 fails to teach how the tele-monitoring service for patients could be aggregated with other services, like for instance safety and security services, telecom services, etc.

Another prior art document, i.e. International Patent Application WO 2007/060396 entitled "Security System and Services" describes a web-based security system that enables a subscriber to register objects. These objects then become labelled with a sticker and so called "good neighbours" are enabled to trigger automated alerts to the subscriber in relation to the registered objects. Again, the communication between the subscribers or "good neighbours" on the one hand and the central register on the other hand is of client-server nature, and the system does not support aggregation of other services than the particular object-related reporting and alerting service.

End-customers today typically consume multiple services delivered by multiple service providers. There is a need for aggregating the services across service provider domains, such that end-customers can seamlessly discover, enable, configure, update and/or disable such services through plug & play and without having to install and maintain dedicated user terminals for each and every service. This need has been recognised for instance in US Patent Application US 2005/0033808 entitled "Application Service Peering and Aggregation", for instance in paragraph [0005] thereof. The architecture disclosed in the latter US patent application however relies on a dedicated server, named SPAS, that proxies the application servers whereto the events are routed. Through the proxies, service applications within or between service providers may become integrated, aggregated or bundled. Proxying the service applications however involves duplication of the application service logic or parts thereof, and consequently has a negative impact on maintenance, memory and processing requirements in the network.

It is an objective of the present invention to provide a system and method for service aggregation in order to overcome the disadvantages and shortcomings of the above identified categories of prior art solutions. More particularly, it is an objective to provide a system and method which enables the end-customer to seamlessly discover, enable, configure, upgrade and disable service using a single, uniform platform through plug & play effortless transactions. It is another objective to avoid installation and use of dedicated, service-specific user terminals for each and every service. It is yet another objective to avoid proxying or duplicating service logic in the network.

### Summary of the Invention

According to the present invention, the above mentioned objectives are realized and the shortcomings of the prior art are overcome by the service aggregation system defined by claim 1. This service aggregation system comprises embedded devices of end-customers, service provisioning infrastructure of plural service providers, a network with connectivity to the embedded devices and connectivity to the service provisioning infrastructure, and a back-end server of a service aggregator, the back-end server having connectivity to the embedded devices via the network and connectivity to the service provisioning infrastructure via the network, and the back-end server being adapted to act and execute business logic commands on each transaction between an embedded device of one of the end-customers and service provisioning infrastructure of one of the service providers, or vice versa.

Thus, instead of a traditional client-server architecture, the present invention introduces a client-back-end-server architecture whereby the aggregating back-end server acts on every transaction between the end-customer and service provider or vice versa. Service providers can register their services with the service aggregator and end-customers can subscribe to new services with the service aggregator. At the customer end, embedded devices are used to connect with the back-end server of the service aggregator. These embedded devices are non-service-specific computing devices with universal connectivity, typically embedded in a modem, set-top-box, PC or stand-alone box. The service providers or service partners connect to the back-end server of the service aggregator through secure links. Thanks to the architecture of the current invention, every customer-provider transaction passes through the service aggregator's back-end infrastructure. As a consequence, the service aggregator offers to the end-customers a uniform platform for enabling, configuring, upgrading, disabling services from multiple service providers. In addition, the service aggregator can create added value for the end-customer by enabling dynamic switching between service providers, offering a uniform billing for services, enabling the end-customer to maintain a profile, etc. Towards the service providers, the service aggregator creates added value in reducing the access threshold for potential customers by removing the necessity to acquire and install dedicated service-specific user terminals, and in providing access to a base of potential subscribers to new services.

In addition to the service aggregation system according to claim 1, the current invention relates to a back end server for use in such a service aggregation system, as defined by claim 12, and a service aggregation method, as defined by claim 13, for aggregating services from plural service providers towards embedded devices of end-customers via a network with connectivity to the embedded devices and connectivity to service provisioning infrastructure of the service providers, whereby the method involves passing each transaction between an embedded device of one of the end-customers and service provisioning infrastructure of one of the service providers, or vice versa, through the back-end server, and executing in the back-end server business logic commands on each transaction between an embedded device of one of said end-customers and service provisioning infrastructure of one of said service providers, or vice versa.

It is noticed that in hindsight of the invention, the operation of web browsers collecting information from different websites and providing the information components in a single window to the user, may show some resemblance with the service aggregation method according to the present invention. United States Patent Application US 2006/0089990 A1 from IBM Corporation, entitled "Method and Apparatus for Relaying Session Information from a Portal Server" for instance shows a system wherein a portal server plays a role in each request from a user for information that has to be collected from plural sources, i.e. plural websites. Upon receipt of a request, portlets, i.e. pieces of code or small applications that run on the portal server, shall collect the content from different websites that has to be embedded into respective portions of the portal page that will be offered in return to the user's request. US 2006/0089990 A1 proposes to relay session information such as the session time out information via the different portlets to the websites in order to have the websites behave consistently. Although the portal in US 2006/0089990 A1 is a single point of access for multiple users to multiple sources of information, there is no execution of business logic commands, i.e. inspection and processing of packets, on each transaction between embedded devices of the end-customers and service provisioning infrastructure of the service providers. Whereas US 2006/0089990 A1 concerns the collection of information for integration in a single portal page that will be displayed to the end-customer, the present invention concerns the aggregation of services that require the management of subscriptions and installation of dedicated hardware.

As is indicated by claim 2, an optional aspect of the system according to the present invention, is that the back-end server may comprise:
a. polling means for regularly receiving poll messages from active embedded devices; and
b. command caching means for caching commands for the embedded devices,
the command caching means and the polling means being operationally coupled to forward cached commands for an embedded device in response to a poll message received from the embedded device.

This way, the back-end server is enabled to penetrate security provisions at the customer side, such as for instance firewalls. An embedded device at the customer end polls the back-end server for instance every minute to verify if a command is cached. The embedded device thereto for instance sends a message containing its identification or serial number to the back-end server. Upon receipt of the polling message, the back-end server will consult its command cache and verify if one or more commands destined to the polling embedded device are stored there. When such command is cached, the polling function in the back-end server will inform the embedded device of that fact, as a result of which the embedded device shall connect with the command cache in the back-end server in order to receive the cached command.

Also optionally, as defined by claim 3, the back-end server in the service aggregation system according to the present invention may comprise:
c. an upload module enabling upload of data files from the embedded devices to the service aggregator's back-end.

The upload module enables transfer of images, moving images and/or large data files from an embedded device to the service aggregator. The upload module might for instance use the HyperText Transfer Protocol Secure (HTTPS) protocol.

Further optionally, as defined by claim 4, the back-end server in the service aggregation system according to the present invention may comprise:
d. a download module enabling download of data files from the service aggregator's back-end to said embedded devices.

Similarly to the upload module, a download module in the back-end server may assist in transferring large files such as multimedia files, images, movies, software packages, etc. from the service aggregator to an embedded device at the customer end. By way of example, the download module could also use the HTTPS protocol.

Further optionally, as defined by claim 5, the said back-end server may comprise:
e. a tunnel server adapted to terminate a first tunnel setup from a first embedded device or from service provisioning infrastructure towards the back-end server, the tunnel server further being operationally coupled with the polling means and the command cache means in order to instruct a second embedded device to setup a second tunnel towards the back-end server, and the tunnel server further being adapted to couple the first tunnel and the second tunnel for secure connectivity between the first embedded device or service provisioning infrastructure and the second embedded device.

This way, in cases where direct communication is needed between two devices, the tunnel server will use the polling function and command cache in order to have one of these devices setup a connection to the back-end server. This connection will be coupled to the connection setup by the second device, which is assumed to take the initiative for the session, towards the back-end server. An example application could for instance be a security service enabling the customer to monitor his house where a camera is hosting a first embedded device, from his office where his PC is hosting a second embedded device. Upon instruction from the customer, the second embedded device shall establish a tunnel towards the tunnel server in the back-end server according to the present invention. In order to be able to penetrate the firewall, the tunnel server shall store a command for the camera to setup a connection and start streaming in the command cache, such that when polling the back-end server, the command will be transferred to the embedded device in the camera. Thereupon the latter embedded device shall setup a second tunnel between the camera and the back-end server. At last, the tunnel server shall couple the two tunnels and establish a secure connection between the first embedded device in the camera and the second embedded device in the office PC whereover real-time images are transferred.

Another option of the service aggregation system according to the present invention, defined by claim 6, is that the back-end server may comprise:
f. business logic adapted to execute commands; and
g. an event router adapted to receive data packets, interpret the header of the data packets, and route the data packets to appropriate business logic in the back-end server.

Thus, the event router will receive packets and address the logic able to interpret, handle or process the packets. The event router thereto interprets the header, e.g. the XML SOAP (extensible Markup Language Simple Object Access Protocol) tag attached to a packet. The business logic will be able to inspect the payload of the packets.

Yet another optional aspect of the current invention is that the back-end server may comprise:
h. a customer portal adapted to maintain status and profile information related to the end-customers;
i. a service aggregator portal adapted to enable a service aggregator to activate, maintain, and deactivate services or service modules; and
j. a service provider portal adapted to enable a service provider to configure its services and to maintain statistics in relation to its services.

Thus, via respective portals, service providers will be able to configure their services in the back-end server of the service aggregator, and get access to statistics such as numbers of transactions, duration of transactions, users of their services, etc. Similarly, via an end-user portal, the users or end-customers will be able to configure their profile, e.g. their interest profile that will be used for advertisement, and be able to monitor status information such as information indicative for the status of their embedded devices. The service aggregator has its own portal to the back-end server in order to be able to activate/de-activate services, activate or de-activate embedded devices, launch new modules, maintain and consult status information and statistics that are either customer related or service related, etc.

Advantageously, as defined by claim 8, the connectivity between the back-end server and the service provisioning infrastructure is realized through one or more encrypted business-to-business links.

Indeed, the connection between the back-end server and the service provider's infrastructure preferably is realized through secure, encrypted business-to-business links, supporting for instance XML over HTTPS.

According to an optional aspect of the present invention defined by claim 9, the connectivity between the embedded devices and the back-end server is realized through standard web services technology.

Indeed, it is noticed that the polling function, command cache, upload module, download module and event router may be implemented as standard web services in the back-end server operating according to the present invention. The communication with those web services will be asynchronous. On the contrary, the communication with the above mentioned tunnel server will be synchronous.

As defined by claim 10, one particular instance of the service aggregation system according to the present invention may use the Internet.

Thus, the secure communication between the customer's embedded device and the back-end server on the one hand, and the secure communication between the back-end server and the service operator infrastructure on the other hand, may be routed through the Internet.

According to a further optional aspect of the present invention service, the back-end server may have a distributed, service-oriented architecture, as defined by claim 11.

Thus, the back-end server can be a distributed system spread over multiple physical locations.

### Brief Description of the Drawings

Fig. 1 illustrates the architecture of an embodiment of the service aggregation system according to the present invention; and
Fig. 2 is a functional block scheme of a preferred embodiment of the back-end server 200 that forms part of the service aggregation system according to the present invention.

### Detailed Description of Embodiment(s)

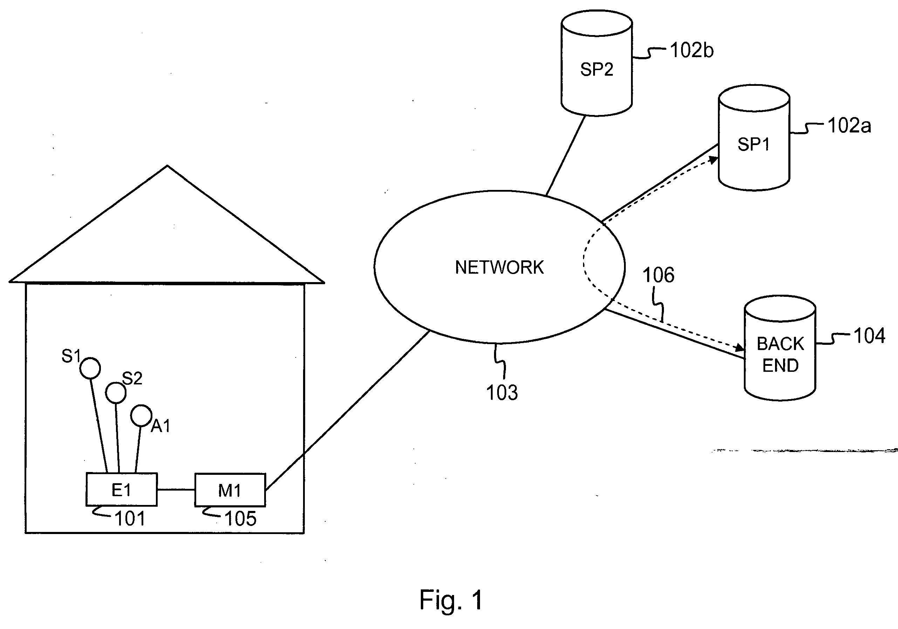

Fig. 1 shows the four discrete parts that form the basis of the service aggregation architecture according to the present invention: the end-customer's embedded device, E1 or 101, the service provider's infrastructure, SP1 or 102a and SP2 or 102b, the service aggregator's back-end server, BACK END or 104, and the communication network, NETWORK or 103, with connectivity to the embedded devices 101, service provider infrastructure 102a and 102b, and back-end aggregation server 104.

In general, the end customer is a user or consumer who wants to consume services from a service partner or service provider 102a or 102b who is connected via the service-aggregation infrastructure 104. Typically, the end customer will be a customer of the service aggregator, e.g. through a subscription, as well as a customer of the service provider which he is interacting with to consume the particular service(s).

The back-end server 104 has a service-oriented architecture, enabling to interface with both the embedded devices 101 at the customer-end as with the service partners 102a and 102b. For every transaction between a customer and a service partner, there is action required of the back-end system 104 in the form of commands executed as part of the business logic inside the back-end server 104.

The service partners 102a and 102b are connected to the aggregating infrastructure using a business-to-business link 106. This link 106 allows them to deliver services seamlessly to their customers over the network.

The network 103 shall typically be an Internet Protocol (IP) based network. In most cases it will be the Internet, but it can also be a private network.

All end-customers are connected via an embedded device like 101 in their house, office, etc. to the service aggregator's back-end server 104. Through this back-end server 104, the service partners or providers 102a and 102b are connected to the end-customers via business-to-business or B2B links like 106. This architecture allows the service partners 102a and 102b to virtually connect directly with the end-customers without having to install their own infrastructure, e.g. a service specific handset or terminal, in the different homes.

The embedded device 101 is connected to the service aggregator's back-end infrastructure 104 through the home network. This home network is typically connected to the Internet 103 via a modem, M1 or 105, and/or router. All communication between the embedded device 101 and the back-end server 104 is preferably fully encrypted. The communication may be wired, wireless or hybrid.

Inside the user's home, one or multiple sensors like S1 and S2, and/or one or multiple actuators like A1 may be connected to the embedded device 101. These I connections can be wired or wireless, depending on the use case of the service or application that will interact with the sensors and/or actuators.

The service partners 102a and 102b are virtually connected to the end-customer's embedded devices 101, but in reality there is only a connection 106 between the service aggregator's back-end 104 and the different service partners 102a and 102b via business-to-business communication. The connection 106 between both parties, 102a or 102b and 104, is established using standard web services over the Internet 103. All communication between the service partners, 102a and 102b, and the aggregator's back-end 104 is also assumed to be fully encrypted.

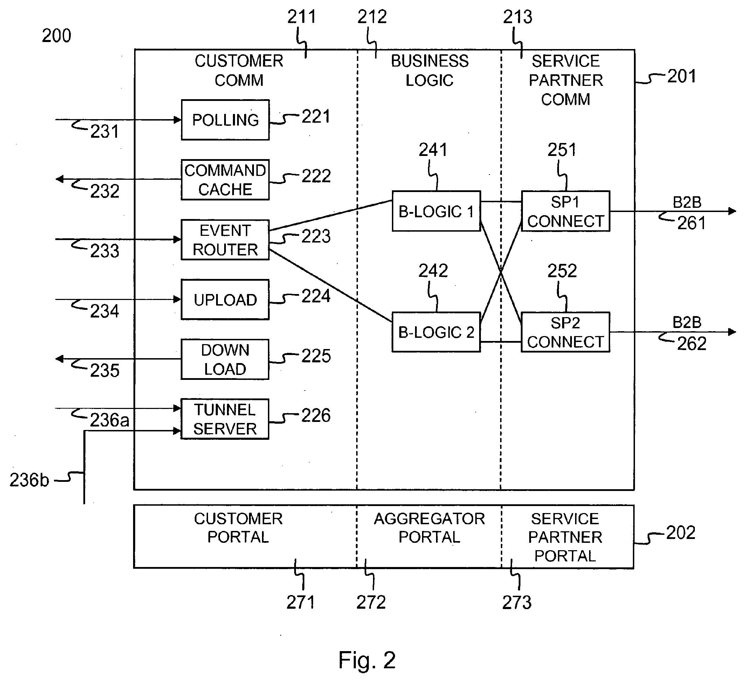

Fig. 2 shows the functional blocks of a particular implementation of the service aggregator's back-end infrastructure 200, and consequently represents one possible instantiation of the back end 104 in Fig. 1. Back end server 200 consists of an operational part 201 holding functionality to interface and communicate with the customer-end denoted by 211, business logic for processing packets denoted by 212, and functionality for interfacing and communicating with the service partners denoted by 213. In addition thereto, the back end server 200 contains a portal 202 that is correspondingly split into a customer portal 271, an aggregator portal 272 and a service partner portal 273. It is noted here that the aggregator portal 272 will represent an administrator portal for the service aggregator.

The customer interface 211 includes a polling function 221 and command cache 222, operationally coupled to establish communication with the embedded device of a customer whenever a command for that embedded device is stored and waiting in the command cache 222. The embedded device shall regularly poll the back end 200, for instance by sending its identification or serial number once every minute. Upon receipt of this message, the polling function 221 shall verify if one or more commands are stored for that embedded device in the command cache 222. In case one or more commands are stored, the embedded device will be instructed to connect with the back end 200, as a result of which a secure connection will be established, penetrating any eventual firewall or other security mechanism at the customer's end. The secure WAN (Wide Area Network) connection established between the embedded device and the back end 200 may for instance be realized using Microsoft's standard .Net, MontaVista, or a proprietary solution. As a result, a secure HTTPS tunnel may be opened between the embedded device and back-end 200.

Assuming that XML over HTTPS is used for communication between the customer's embedded devices and the back end 200, the event router 223 shall receive XML messages, inspect the header thereof, and for instance depending on the contents of the XML SOAP tag, deciding to which business logic the message will be forwarded for further handling. In Fig. 2 this is illustrated by the connection between event router 223 and respectively first business logic 241 and second business logic 242. The business logic shall inspect and process the payload of the XML messages.

HTTPS upload module 224 enables to upload large data or media files from the customer to the back-end 200 over a connection 234 with substantially larger capacity than the connections 231 and 233 that are respectively used to convey the polling messages and event-related messaging from embedded devices.

Similarly, HTTPS download module 225 enables to download large data or media files from the back-end 200 to the customer's embedded device over a connection 235 with substantially larger capacity than the connection 232 that is used to convey commands from the back-end 200 to the embedded device.

The tunnel server 226 at last enables direct communication between an embedded device at the customer end and for instance another embedded device residing with another customer, a service partner, or at a different location like for instance the customer's work office. An incoming tunnel 236b can for instance be established from the customer's work office PC towards the tunnel server 226 in case the customer has subscribed to a security service enabling the customer to remotely activate one or more cameras and monitor certain rooms in his house. The tunnel server is operationally coupled to the commend cache 222 and polling function 221, and shall enter a command for activation of the camera in the command cache 222. At the first instance where the embedded device located at the customer's house polls the back-end 200, the polling function 221 and command cache 222 will forward the stored command instructing activation of the camera to the customer's embedded device. Thereupon, the latter embedded device shall setup a second tunnel 236a towards the tunnel server 226, and the tunnel server 226 shall couple the first and second tunnel in order to enable direct streaming of the camera-recorded images from the customer's house to the customer's work office PC through a secure HTTPS connection.

It is noted that although the tunnel server operation has been explained here above by way of an example application, i.e. remote monitoring of certain rooms in the customer's house, it is clear that presence of a tunnel server in a service aggregation back-end according to the present invention could be of interest whenever services may require direct communication between embedded devices. Such direct communication needs to surpass the firewalls and therefore can be established only by means of a tunnel server operating in conjunction with a polling function and command cache in such a manner that the embedded devices that do not take the initiative for the communication are instructed to setup an outgoing tunnel towards the tunnel server. In a second instance, that outgoing tunnel will be coupled by the tunnel server to an incoming tunnel setup by the embedded device taking the initiative for the communication.

At the service partner side 213, the back end 200 hosts interfaces to each service provider. In Fig. 2, a first interface 251 towards a first service provider SP1 is shown, and a second interface 252 towards a second service provider SP2 is shown. Standard web service communication over business-to-business links 261 and 262 is used in order to forward event related messages to the service providers. As an example, XML over HTTPS may be used over the B2B links 261 and 262, although it is not excluded that other types of messaging like for instance e-mail messages are used instead of XML if for example a restaurant reservation service is supported through the aggregation back end 200.

In the following paragraphs, the operation of the service aggregation system according to the present invention will be illustrated by an example, i.e. a self-monitoring and service partner monitoring service.

An end-customer can register for a service, in this example the self-monitoring or service partner monitoring service, by entering specific details about himself and his environment in the customer portal 271 hosted by the aggregator's back end 200. Through the portal interface, the customer links specific actuators (like for instance a pushbutton) with specific sensors (like for instance a camera). The customer links the actuators to enable a scene, i.e. a specific set of sensors which become active to detect motion. In addition, the customer can specify whether sensor events need to be routed to him, e.g. by email, SMS, MMS or other means of communication, or need to be routed to the service partner he has subscribed himself to. Routing to the service partner will pass through a B2B web services link. At any point in time, the customer can change his profile to route the event to him only, to a service provider or to both, and the customer can change links between actuators and sensors to enable different scenes.

When the sensor, in this case a camera, is active and detects motion it will send an event through the network, i.e. the Internet, to the service aggregator's back-end 200. This back-end 200 will use the pre-configured business logic to route the information to the customer and/or to the service provider SP1, depending on the service details. Either the service provider SP1 or the customer will login into the portal to see the event details and see the current state of the sensors. In this example, they may connect to the cameras to get live video data. The customer and/or the service provider will take the necessary actions depending on the sensor data he is consuming.

Whereas the customer portal 271 enables the user to configure his profile, the aggregator portal 272 is an administrator portal foreseen to enable the service aggregator to activate/de-activate new services, to activate/de-activate embedded devices, to maintain status and statistics, etc. Further, a service partner portal 273 is hosted by the back-end 200 in order to enable a service partner that has subscribed with the service aggregator to configure his services, and to access certain statistics like amounts of transactions, and statistics related to the users of his services.

Obviously, the services that can be supported through a service aggregation platform with architecture according to the present invention are manifold. Security and safety related services, reservation services, telecom related services, telemedicine services, shopping, entertainment, etc. can all run through a single platform that does not require complex user terminals like a PC or a service specific terminal, but simply relies on remotely chargeable and controllable always-on embedded devices with universal connectivity (USB, Bluetooth, WiFi, UMTS, Wireless USB, HDMi, etc.). The platform enables to dynamically switch between service providers. The end-user only needs a web browser, e.g. via his TV set, to log into the customer portal of the service aggregator's back-end infrastructure, or - in case of patients, kids, elderly people, ... - the end-user may rely on an assistant, care-giver, parent, etc. in order to configure the services he desires to use through the platform.

Although the present invention has been illustrated by reference to specific embodiments, it will be apparent to those skilled in the art that the invention is not limited to the details of the foregoing illustrative embodiments, and that the present invention may be embodied with various changes and modifications without departing from the scope thereof. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein. In other words, it is contemplated to cover any and all modifications, variations or equivalents that fall within the spirit and scope of the basic underlying principles and whose essential attributes are claimed in this patent application. It will furthermore be understood by the reader of this patent application that the words "comprising" or "comprise" do not exclude other elements or steps, that the words "a" or "an" do not exclude a plurality, and that a single element, such as a computer system, a processor, or another integrated unit may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. It is to be understood that the terms so used are interchangeable under appropriate circumstances and embodiments of the invention are capable of operating according to the present invention in other sequences different from the one(s) described or illustrated above.

## Claims

1. A service aggregation system comprising embedded devices (101) of end-customers, service provisioning infrastructure (102a, 102b) of plural service providers, and a network (103) with connectivity to said embedded devices (101) and connectivity to said service provisioning infrastructure (102a, 102b),
said service aggregation system further comprises a back-end server (104, 200) of a service aggregator, said back-end server (104, 200) having connectivity to said embedded devices (101) via said network (103) and connectivity (106) to said service provisioning infrastructure (102a, 102b) via said network (103), **CHARACTERIZED IN THAT** said back-end server (104, 200) being adapted to act and execute business logic commands on each transaction between an embedded device (101) of one of said end-customers and service provisioning infrastructure (102a, 102b) of one of said service providers, or vice versa.

2. A service aggregation system according to claim 1,
**CHARACTERIZED IN THAT** said back-end server (104, 200) comprises:
a. polling means (221) for regularly receiving poll messages from active embedded devices (101); and
b. command caching means (222) for caching commands for said embedded devices (101),
said command caching means (222) and said polling means (221) being operationally coupled to forward cached commands for an embedded device (101) in response to a poll message received from said embedded device (101).

3. A service aggregation system according to claim 1,
**CHARACTERIZED IN THAT** said back-end server (104, 200) comprises:
c. an upload module (224) enabling upload of data files from said embedded devices (101) to said back-end server (104, 200).

4. A service aggregation system according to claim 1,
**CHARACTERIZED IN THAT** said back-end server (104, 200) comprises:
d. a download module (225) enabling download of data files from said back-end server (104, 200) to said embedded devices (101).

5. A service aggregation system according to claim 1,
**CHARACTERIZED IN THAT** said back-end server (104, 200) comprises:
e. a tunnel server (226) adapted to terminate a first tunnel (236b) setup from a first embedded device or from service provisioning infrastructure towards said back-end server (104, 200), said tunnel server (226) further being operationally coupled with said polling means (221) and said command cache means (222) in order to instruct a second embedded device to setup a second tunnel (236a) towards said back-end server (104, 200), and said tunnel server (226) further being adapted to couple said first tunnel (236b) and said second tunnel (236a) for secure connectivity between said first embedded device or service provisioning infrastructure and said second embedded device.

6. A service aggregation system according to claim 1,
**CHARACTERIZED IN THAT** said back-end server (104, 200) comprises:
f. business logic (241, 242) adapted to execute commands; and
g. an event router (223) adapted to receive data packets, interpret overhead of said data packets, and route said data packets to appropriate business logic (241, 242) in said back-end server.

7. A service aggregation system according to claim 1,
**CHARACTERIZED IN THAT** said back-end server (104, 200) comprises:
h. a customer portal (271) adapted to maintain status and profile information related to said end-customers;
i. a service aggregator portal (272) adapted to enable a service aggregator to activate, maintain, and deactivate services or service modules; and
j. a service provider portal (273) adapted to enable a service provider to configure its services and to maintain statistics in relation to its services.

8. A service aggregation system according to claim 1,
**CHARACTERIZED IN THAT** said connectivity (106) between said back-end server (104, 200) and said service provisioning infrastructure (102a, 102b) is realized through one or more encrypted business-to-business links.

9. A service aggregation system according to claim 1,
**CHARACTERIZED IN THAT** said connectivity between said embedded devices (101) and said back-end server (104, 200) is realized through standard web services technology.

10. A service aggregation system according to claim 1,
**CHARACTERIZED IN THAT** said network (103) is the Internet.

11. A service aggregation system according to claim 1,
**CHARACTERIZED IN THAT** said back-end server (104, 200) has a distributed, service-oriented architecture.

12. A back end server (104, 200) for use in a service aggregation system as defined in any one of the preceding claims.

13. A service aggregation method for aggregating services from plural service providers towards embedded devices (101) of end-customers via a network (103) with connectivity to said embedded devices (103) and connectivity to service provisioning infrastructure (102a, 102b) of said service providers,
said service aggregation method involves passing each transaction between an embedded device (101) of one of said end-customers and service provisioning infrastructure (102a, 102b) of one of said service providers, or vice versa, through a back-end server (104, 200), and **CHARACTERIZED IN THAT** executing in said back-end server (104, 200) business logic commands on each transaction between an embedded device (101) of one of said end-customers and service provisioning infrastructure (102a, 102b) of one of said service providers, or vice versa.

## Patentansprüche

1. Dienstaggregationssystem, umfassend integrierte Vorrichtungen (101) von Endkunden, eine Diensterbringungsinfrastruktur (102a, 102b) mehrerer Diensterbringer sowie ein Netzwerk (103) mit Anbindung an die integrierten Vorrichtungen (101) und Anbindung an die Diensterbringungsinfrastruktur (102a, 102b),
wobei das Dienstaggregationssystem ferner einen Back-End-Server (104, 200) eines Dienstaggregators umfasst, wobei der Back-End-Server (104, 200) Anbindung an die integrierten Vorrichtungen (101) über das Netzwerk (103) und Anbindung (106) an die Diensterbringungsinfrastruktur (102a, 102b) über das Netzwerk (103) aufweist, **dadurch gekennzeichnet, dass** der Back-End-Server (104, 200) zum Betätigen und Ausführen von Geschäftslogikbefehlen bei jeder Transaktion zwischen einer integrierten Vorrichtung (101) von einem der Endkunden und einer Diensterbringungsinfrastruktur (102a, 102b) von einem der Diensterbringer, oder umgekehrt, ausgelegt ist.

2. Dienstaggregationssystem nach Anspruch 1,
**dadurch gekennzeichnet, dass** der Back-End-Server (104, 200) Folgendes umfasst:
a. Abrufmittel (221) zum regelmäßigen Empfangen von Abrufnachrichten von aktiven integrierten Vorrichtungen (101); und
b. Befehlzwischenspeicherungsmittel (222) zum Zwischenspeichern von Befehlen für die integrierten Vorrichtungen (101),
wobei das Befehlzwischenspeicherungsmittel (222) und das Abrufmittel (221) betriebsfähig gekoppelt sind, um zwischengespeicherte Befehle für eine integrierte Vorrichtung (101) als Antwort auf eine von der integrierten Vorrichtung (101) empfangene Abrufnachricht weiterzuleiten.

3. Dienstaggregationssystem nach Anspruch 1,
**dadurch gekennzeichnet, dass** der Back-End-Server (104, 200) Folgendes umfasst:
c. ein Hochlademodul (224), das Hochladen von Dateien von den integrierten Vorrichtungen (101) auf den Back-End-Server (104, 200) gestattend.

4. Dienstaggregationssystem nach Anspruch 1,
**dadurch gekennzeichnet, dass** der Back-End-Server (104, 200) Folgendes umfasst:
d. ein Herunterlademodul (225), das Herunterladen von Dateien von dem Back-End-Server (104, 200) auf die integrierten Vorrichtungen (101) gestattend.

5. Dienstaggregationssystem nach Anspruch 1,
**dadurch gekennzeichnet, dass** der Back-End-Server (104, 200) Folgendes umfasst:
e. einen Tunnelserver (226), zum Bestimmen einer ersten Tunnel- (236b) Einrichtung von einer ersten integrierten Vorrichtung oder von einer Diensterbringungsinfrastruktur an den Back-End-Server (104, 200) ausgelegt, wobei der Tunnelserver (226) ferner betriebsfähig mit dem Abrufmittel (221) und dem Befehlzwischenspeicherungsmittel (222) gekoppelt ist, um eine zweite integrierte Vorrichtung anzuweisen, einen zweiten Tunnel (236a) an den Back-End-Server (104, 200) einzurichten, und wobei der Tunnelserver (226) ferner zum Koppeln des ersten Tunnels (236b) und des zweiten Tunnels (236a) für eine sichere Anbindung zwischen der ersten integrierten Vorrichtung oder Diensterbringungsinfrastruktur und der zweiten integrierten Vorrichtung ausgelegt ist.

6. Dienstaggregationssystem nach Anspruch 1,
**dadurch gekennzeichnet, dass** der Back-End-Server (104, 200) Folgendes umfasst:
f. eine zum Ausführen von Befehlen ausgelegte Geschäftslogik (241, 242); und
g. einen Ereignisrouter (223), zum Empfangen von Datenpaketen, Interpretieren von Kopfdaten der Datenpakete und Leiten der Datenpakete an eine geeignete Geschäftslogik (241, 242) in dem Back-End-Server ausgelegt.

7. Dienstaggregationssystem nach Anspruch 1,
**dadurch gekennzeichnet, dass** der Back-End-Server (104, 200) Folgendes umfasst:
h. ein Kundenportal (271), zum Pflegen von mit den Endkunden verbundenen Status- und Profilinformationen aufgelegt;
i. ein Dienstaggregatorportal (272), dazu ausgelegt, einem Dienstaggregator zu gestatten, Dienste oder Dienstmodule zu aktivieren, pflegen und deaktivieren; und
j. ein Diensterbringerportal (273), dazu ausgelegt, einem Diensterbringer zu gestatten, seine Dienste zu konfigurieren und Statistiken in Bezug auf seine Dienste zu pflegen.

8. Dienstaggregationssystem nach Anspruch 1,
**dadurch gekennzeichnet, dass** die Anbindung (106) zwischen dem Back-End-Server (104, 200) und der Diensterbringungsinfrastruktur (102a, 102b) durch eine oder mehrere verschlüsselte Geschäft-zu-Geschäft-Verbindungen ausgeführt wird.

9. Dienstaggregationssystem nach Anspruch 1,
**dadurch gekennzeichnet, dass** die Anbindung zwischen den integrierten Vorrichtungen (101) und dem Back-End-Server (104, 202) durch übliche Webservice-Technologie umgesetzt wird.

10. Dienstaggregationssystem nach Anspruch 1,
**dadurch gekennzeichnet, dass** das Netzwerk (103) das Internet ist.

11. Dienstaggregationssystem nach Anspruch 1,
**dadurch gekennzeichnet, dass** der Back-End-Server (104, 200) eine verteilte, dienstorientierte Architektur umfasst.

12. Back-End-Server (104, 200) zur Verwendung in einem Dienstaggregationssystem nach einem der vorhergehenden Ansprüche.

13. Dienstaggregationsverfahren zum Aggregieren von Diensten von mehreren Diensterbringern an integrierte Vorrichtungen (101) von Endkunden über ein Netzwerk (103) mit Anbindung an die integrierten Vorrichtungen (103) [sic.] und Anbindung an die Diensterbringungsinfrastruktur (102a, 102b) der Diensterbringer,
wobei das Dienstaggregationsverfahren das Weiterleiten jeder Transaktion zwischen einer integrierten Vorrichtung (101) von einem der Endkunden und einer Diensterbringungsinfrastruktur (102a, 102b) von einem der Diensterbringer, oder umgekehrt, durch einen Back-End-Server (104, 200) umfasst, und **dadurch gekennzeichnet, dass** in dem Back-End-Server (104, 200) Geschäftslogikbefehle bei jeder Transaktion zwischen einer integrierten Vorrichtung (101) von einem der Endkunden und einer Diensterbringungsinfrastruktur (102a, 102b) von einem der Diensterbringer, oder umgekehrt, ausgeführt werden.

## Revendications

1. Système d'agrégation de services comprenant des dispositifs intégrés (101) de clients finaux, des infrastructures de fourniture de services (102a, 102b) d'une pluralité de fournisseurs de services, et un réseau (103) avec une connectivité avec lesdits dispositifs intégrés (101) et une connectivité avec ladite infrastructure de fourniture de services (102a, 102b),
ledit système d'agrégation de services comprenant en outre un serveur dorsal (104, 200) d'un agrégateur de services, ledit serveur dorsal (104, 200) étant en connectivité avec lesdits dispositifs intégrés (101) via ledit réseau (103) et en connectivité (106) avec ladite infrastructure de fourniture de services (102a, 102b) via ledit réseau (103), **caractérisé en ce que** ledit serveur dorsal (104, 200) est adapté pour agir et exécuter des commandes logique d'affaires sur chaque transaction entre un dispositif intégré (101) de l'un desdits clients finaux et l'infrastructure de fourniture de services (102a, 102b) de l'un desdits fournisseurs de services, ou vice versa.

2. Système d'agrégation de services selon la revendication 1,
**caractérisé en ce que** ledit serveur dorsal (104, 200) comprend:
a. des moyens d'interrogation (221) destiné à recevoir régulièrement des messages d'interrogation provenant de dispositifs intégrés actifs (101) ; et
b. des moyens de prise de commandes (222) destinés à prendre des commandes destinées auxdits dispositifs intégrés (101),
lesdits moyens de prise de commandes (222) et lesdits moyens d'interrogation (221) étant éventuellement couplés pour transmettre des commandes prises destinées à un dispositif intégré (101) en réponse à un message d'interrogation reçu dudit dispositif intégré (101).

3. Système d'agrégation de services selon la revendication 1, **caractérisé en ce que** ledit serveur doral (104, 200) comprend:
c. un module de télétransmission (224) permettant de télétransmettre des fichiers de données desdits dispositifs intégrés (101) vers ledit serveur doral (104, 200).

4. Système d'agrégation de services selon la revendication 1, **caractérisé en ce que** ledit serveur doral (104, 200) comprend:
d. un module de téléchargement (225) permettant de télécharger des fichiers de données dudit serveur doral (104, 200) vers lesdits dispositifs intégrés (101).

5. Système d'agrégation de services selon la revendication 1,
**caractérisé en ce que** ledit serveur doral (104, 200) comprend:
e. un serveur de tunnel (226) adapté pour mettre fin à un premier tunnel (236b) mis en place d'un premier dispositif intégré ou une première infrastructure de fourniture de services jusqu'audit serveur doral (104, 200), ledit serveur de tunnel (226) étant en outre couplé de manière opérationnelle auxdits moyens d'interrogation (221) et auxdits moyens de prise de commande (222) afin de charger un second dispositif intégré de mettre en place un second tunnel (236a) jusqu'audit serveur doral (104, 200), et ledit serveur de tunnel (226) étant en outre adapté pour coupler ledit premier tunnel (236b) et ledit second tunnel (236a) pour établir une connectivité sécurisée entre ledit premier dispositif intégré ou ladite première infrastructure de fourniture de services et ledit second dispositif intégré.

6. Système d'agrégation de services selon la revendication 1,
**caractérisé en ce que** ledit serveur doral (104, 200) comprend:
f. une logique d'affaires (241, 242) adapter pour exécuter des commandes; et
g. un routeur d'événement (223) adapté pour recevoir des paquets de données, interpréter l'en-tête desdits paquets de données, et acheminer lesdits paquets de données à la logique d'affaires (241, 242) appropriée dans ledit serveur doral.

7. Système d'agrégation de services selon la revendication 1, **caractérisé en ce que** ledit serveur doral (104, 200) comprend:
h. un portail client (271) adapté pour maintenir des informations de statut et de profil relatives à ces clients finaux;
i. un portail d'agrégateur de services (272) adapté pour permettre à un agrégateur de services d'activer, de maintenir et de désactiver des services ou des modules de service ; et
j. un portail de fournisseurs de services (273) adapté pour permettre à un fournisseur de services de configurer ses services et de tenir des statistiques en rapport avec ses services.

8. Système d'agrégation de services selon la revendication 1,
**caractérisé en ce que** ladite connectivité (106) entre ledit serveur doral (104, 200) et ladite infrastructure de fourniture de service (102a, 102b) est réalisée par le biais d'un ou de plusieurs liaisons business-to-business cryptés.

9. Système d'agrégation de services selon la revendication 1,
**caractérisé en ce que** ladite connectivité entre lesdits dispositifs intégrés (101) et ledit serveur dorsal (104, 200) est réalisée grâce à la technologie de services web standard.

10. Système d'agrégation de services selon la revendication 1,
**caractérisé en ce que** ledit réseau (103) est l'Internet.

11. Système d'agrégation de services selon la revendication 1, **caractérisé en ce que** ledit serveur doral (104, 200) a une architecture distribuée orientée services.

12. Serveur doral (104, 200) destiné à être utilisé dans un système d'agrégation de services tel que défini dans l'une quelconque des revendications précédentes.

13. Procédé d'agrégation de service pour agréger des services depuis une pluralité de fournisseurs de services vers des dispositifs intégrés (101) de clients finaux via un réseau (103) en connectivité avec lesdits dispositifs intégrés (103) et en connectivité avec l'infrastructure de fourniture de services (102a, 102b) desdits fournisseurs de services,
ledit procédé d'agrégation de services consistant à faire passer chaque transaction entre un dispositif intégré (101) de l'un desdits clients finaux et une infrastructure de fourniture de services (102a, 102b) de l'un desdits fournisseurs de services, ou vice versa, par le biais d'un serveur doral (104, 200), et **caractérisé par** l'exécution dans ledit serveur doral (104, 200) de commandes de logique d'affaires sur chaque transaction entre un dispositif intégré (101) de l'un desdits clients finaux et une infrastructure de fourniture de services (102a, 102b) de l'un desdits fournisseurs de services, ou vice versa.
